# EUROPEAN PATENT APPLICATION

(11) **EP 2 573 558 A1**
(43) Date of publication of application: **27.03.2013**
(21) Application number: 11182484.3
(22) Date of filing: 23.09.2011
(51) Int. Cl.: G01N 33/50

(54) **Modulation of G protein-mediated signal transduction pathways**

(71) Applicant: Philip Morris Products S.A., 2000 Neuchâtel (CH)
(72) Inventor: Lammerich, Hans-Peter, 53773 Hennef (DE)
(74) Representative: Vossius & Partner

(57) **Abstract**

The present invention relates to methods for regulating signal transduction pathways activated by cigarette smoke by modulating, particularly by agonizing or antagonizing, the function and/or activity of a GPCR, which is directly modulated by cigarette smoke. The invention further relates to methods for assessing cigarette smoke or fractions thereof which directly modulates the activity of a GPCR..

## Description

The present invention relates to methods for regulating signal transduction pathways activated by cigarette smoke by modulating, particularly by agonizing or antagonizing, the function and/or activity of a G-Protein Coupled Receptor (GPCR), which is directly modulated by cigarette smoke. The invention further relates to compounds that can be used in such a method and may further be useful for studying cigarette smoke-associated diseases or disorders.

GPCRs are the largest gene family in the human genome. More than 50% of drugs on the market are either agonists or antagonists on GPCRs (Im, 2002). Positive or negative modulation of GPCRs with drugs has turned out to be successful tools for the treatment of many diseases such as allergy, gastric ulcer, and hypertension and others. GPCRs are anchored in the cell membrane and the structural feature common to all GPCRs is a seven-helical transmembrane domain. GPCR activation is evoked by stimuli as diverse as light, Ca2+, odorants, amino acids, nucleotides, proteins, polypeptides, steroids, and fatty acid derivatives.

The completion of the human genome project has identified about 865 potential GPCR genes (Perez, 2005). Apart from the group of sensory receptors, 367 GPCRs have been considered as potential receptors for endogenous ligands in the human genome based on DNA sequence homologies (Vassilatis et al, 2003). Since about 150 of these potential receptors still await chemical identification of their ligands, they are marked as 'orphan receptors'. (Im, 2002; Kostenis, 2004).

Whitney et al (2003) have described a GPCR family member RAI-3 that is phosphorylated, and associated with tyrosine phosphorylated activation complexes, following exposure of cells to cigarette smoke.

Up to now the sensor/receptor for cigarette smoke at the cell surface is not known. Identification of such a sensor/receptor would provide the possibility for examining the cause-and-effect relationship between the cell surface sensor/receptor, and smoking and allow for a better understanding of the risk of smoking. The receptor and its associated downstream signal transduction pathway would further provide a source of materials for research in smoking-related disease and molecular targets for intervention.

There was therefore a need for identifying the sensor/receptor for cigarette smoke at the cell surface.

This need was met within the scope of the present invention.

The results presented herein show that a number of GPCR members on the cell surface, but particularly the GPR109A polypeptide, are directly activated by substances present in cigarette smoke. GPR109A is expressed in white and brown adipose tissue (Lorenzen et al., 2002). Lower expression was detected in spleen and lung tissue, and little to no expression was detected in all other tissues examined (Wise et al., 2003). It was further demonstrated that the murine PUMA-G (Protein Upregulated in Macrophages by interferon-Gamma) and its human ortholog HM74A are receptors for nicotinic acid (collectively called GPR109A). The nicotinic acid receptor expressed on mouse macrophages was shown to have pharmacological properties indistinguishable from the G-protein-coupled nicotinic acid receptor expressed on spleen cell membranes (Lorenzen et al., 2002). It was thus suggested that the effects of nicotinic acid on macrophages, spleen, and adipocytes are mediated via an identical G-protein-coupled receptor (Tunaru et al., 2003, Wise et al., 2003). GPR109A also acts as a high affinity receptor for (D)-beta-hydroxybutyrate and mediates increased adiponectin secretion and decreased lipolysis through G(i)-protein-mediated inhibition of adenylyl cyclase. This pharmacological effect requires nicotinic acid doses that are much higher than those provided by a normal diet. GPR109A mediates nicotinic acid-induced apoptosis in mature neutrophils. Receptor activation by nicotinic acid results in reduced cAMP levels which may affect activity of cAMP-dependent protein kinase A and phosphorylation of target proteins, leading to neutrophil apoptosis. Due to the anti-lipolytic effect, niacin is used for treatment of atherosclerosis. As an unwanted side effect, Niacin elicits the release of prostaglandin D2 from Langerhans cells which causes a marked local cutaneous vasodilation, also called flushing (Maciejewski-Lenoir et al, 2006).

The GPCR members identified herein may thus be used, for example, in a method of screening compounds known to be present in any aerosol generated by heating or combusting tobacco, referred to herein generally as smoke which includes but is not limited to cigarette smoke, for receptor-modulating activity. In a specific embodiment, said method comprises fractionating the constituents of cigarette smoke, particularly the constituents of the total particulate matter or the gas vapour phase of cigarette smoke, and testing the various fractions for receptor-modulating activity. Further, compounds identified in such a screening method or compounds already known to have receptor-modulating activity on the receptor identified herein may be used for verifying the health effects caused by cigarette smoke with the aim of finding alternative means for the treatment, prevention or amelioration of cigarette smoke associated diseases.

In one embodiment, the present invention thus now provides a method for regulating signal transduction pathways which are activated by cigarette smoke, which method comprises modulating, particularly agonizing or antagonizing, the function and/or activity of a GPCR member on the cell surface, which is directly activated by one or more substances present in an aerosol generated by heating or combusting tobacco such as cigarette smoke. In a specific embodiment, said GPCR member is selected from the group consisting of 5-HT2B, APJ, A2B, CB1, CCR10, CCR4, CGRP1, CXCR1, D1, D2, D4, EP3, EP4, ETA, GAL1, GPR40, GPR68, H1, H3, M2, MC4, mGluR2, NMU2, OPRK1, OPRM1, P2Y11, S1P4, sst3, VPAC2, Y2, GPR109A and S1P4, but particularly said GPCR member is the GPR109A polypeptide.

In a specific embodiment of the invention, a method is provided for regulating signal transduction pathways activated by cigarette smoke, particularly by the total particulate matter (TPM) present in cigarette smoke, which method comprises antagonizing the function and/or activity of the GPR109A polypeptide on the surface of cells which are exposed to cigarette smoke or to a constituent of cigarette smoke, particularly one or more substances present in the total particulate matter or the gas vapour phase of cigarette smoke.

GPR109A expression in immune cells appears to be regulated by various cytokines. GPR109A expression can be upregulated by GM-CSF in neutrophils (Yousefi et al, 2000), whereas IFNγ increases GPR109A expression in monocytes, and macrophages (Schaub et al, 2001). Knowles et al. (2006) demonstrates that human macrophage cell lines and primary cells in culture express both HM74 (GPR109B) and HM74a (GPR109A) and that these receptors can directly mediate macrophage responses to niacin via activation of the prostaglandin synthesis pathway and via increased expression and activity of PPARγ. Szanto and Roszer (2008) reviewed the role of PPARγ in macrophages and the involvement of PPARγ in artherosclerosis. On one hand PPARγ enhances macrophage/foam cell development and on the other hand it inhibits expression of molecules involved in macrophage chemotaxis.

In one embodiment of the invention, a method is provided for regulating or modulating the prostaglandin synthesis pathway, which method comprises modulating, particularly agonizing or antagonizing, the function and/or activity of a GPCR member on the cell surface, which is directly activated by one or more substances present in an aerosol generated by heating or combusting tobacco such as cigarette smoke. In a specific embodiment, said GPCR member is selected from the group consisting of 5-HT2B, APJ, A2B, CB1, CCR10, CCR4, CGRP1, CXCR1, D1, D2, D4, EP3, EP4, ETA, GAL1, GPR40, GPR68, H1, H3, M2, MC4, mGluR2, NMU2, OPRK1, OPRM1, P2Y11, S1P4, sst3, VPAC2, Y2, GPR109A and S1P4, but particularly said GPCR member is the GPR109A polypeptide.

In a specific embodiment of the invention, a method is provided for regulating or modulating neutrophil apoptosis that is activated by an aerosol generated by heating or combusting tobacco such as cigarette smoke, particularly by the total particulate matter (TPM) present in cigarette smoke, which method comprises antagonizing the function and/or activity of the GPR109A polypeptide on the surface of cells which are exposed to cigarette smoke or to a constituent of cigarette smoke, particularly one or more substances present in the total particulate matter or the gas vapour phase of cigarette smoke.

In a specific embodiment of the invention, a method is provided for regulating or modulating the prostaglandin synthesis pathway activated by an aerosol generated by heating or combusting tobacco such as cigarette smoke, particularly by the total particulate matter (TPM) present in cigarette smoke, which method comprises antagonizing the function and/or activity of the GPR109A polypeptide on the surface of cells which are exposed to cigarette smoke or to a constituent of cigarette smoke, particularly one or more substances present in the total particulate matter or the gas vapour phase of cigarette smoke.

This regulation or modulation of signal transduction pathways activated by an aerosol generated by heating or combusting tobacco such as cigarette smoke, particularly by the total particulate matter (TPM) present in cigarette smoke, comprising agonizing or antagonizing the function and/or activity of a GPCR member on the cell surface, which is directly modulated by cigarette smoke or a constituent of cigarette smoke, particularly one or more substances present in the total particulate matter or the gas vapour phase of cigarette smoke, now offers new options for the treatment of diseases or disorders which are directly or indirectly associated with or induced by the exposure to cigarette smoke. In a specific embodiment of the invention, said GPCR member is a GPR409A polypeptide. For example, the signal transduction pathways affected by agonizing GPR109A include reduced cAMP levels, reduced activity of cAMP-dependent protein kinase A and reduced phosphorylation of target proteins.

The present invention thus further relates, in one embodiment, to a method for studying the effects associated with or induced by exposure to an aerosol generated by heating or combusting tobacco such as cigarette smoke in an individual comprising administering to said individual an effective amount of a compound capable of modulating, particularly of agonizing or antagonizing, the function and/or activity of a GPCR member on the surface of cells, particularly of cells which are exposed to cigarette smoke such as cells present in lung tissue, which GPCR member is directly activated by substances present in the smoke of tobacco burning material such as cigarette smoke. In a specific embodiment, said GPCR member is selected from the group consisting of 5-HT2B, APJ, A2B, CB1, CCR10, CCR4, CGRP1, CXCR1, D1, D2, D4, EP3, EP4, ETA, GAL1, GPR40, GPR68, H1, H3, M2, MC4, mGluR2, NMU2, OPRK1, OPRM1, P2Y11, S1P4, sst3, VPAC2, Y2, GPR109A and S1P4, particularly said GPCR member is a GPR409A polypeptide.

As a result of the above method new options for the treatment, prevention or amelioration of a disease, disorder or condition associated with or induced by exposure to cigarette smoke in an individual in need of such a treatment are contemplated comprising administering to said individual a therapeutically effective amount of a compound capable of modulating, particularly of antagonizing, the function and/or activity of a GPR109A.

In one embodiment, the present invention provides a compound that is capable of modulating the function and/or activity of a GPCR member on the surface of a cell, particularly a cell such as a cell present in lung tissue, exposed to an aerosol generated by heating or combusting tobacco, e.g., cigarette smoke, which GPCR member is directly modulated by cigarette smoke or a constituent of cigarette smoke, particularly one or more substances present in the total particulate matter or the gas vapour phase of cigarette smoke In a specific embodiment, said GPCR member is selected from the group consisting of 5-HT2B, APJ, A2B, CB1, CCR10, CCR4, CGRP1, CXCR1, D1, D2, D4, EP3, EP4, ETA, GAL1, GPR40, GPR68, H1, H3, M2, MC4, mGluR2, NMU2, OPRK1, OPRM1, P2Y11, S1P4, sst3, VPAC2, Y2, GPR109A and S1 P4.

In a specific embodiment of the invention, said GPCR member is the GPR109A polypeptide.

In one embodiment, said compound is an agonist of GPCR activity.

In one embodiment, said compound is an antagonist of GPCR activity.

Diseases, disorders or conditions associated with or induced by exposure to cigarette smoke that can be studied by the method according to the present invention includes, without being limited thereto, lung diseases such as chronic obstructive pulmonary disease (COPD) (including emphysema and chronic bronchitis), heart diseases such as coronary artery disease, heart attacks, strokes, and cancer, particularly acute myeloid leukemia; bladder cancer, cancer of the cervix, cancer of the esophagus, kidney cancer, cancer of the larynx (voice box), lung cancer, cancer of the oral cavity (mouth), cancer of the pharynx (throat), stomach cancer, cancer of the uterus, and peripheral vascular diseases and hypertension, arteriosclerosis.

In one embodiment, the present invention relates to the use of a GPCR as identified herein, particularly a GPCR selected from the group consisting of 5-HT2B, APJ, A2B, CB1, CCR10, CCR4, CGRP1, CXCR1, D1, D2, D4, EP3, EP4, ETA, GAL1, GPR40, GPR68, H1, H3, M2, MC4, mGluR2, NMU2, OPRK1, OPRM1, P2Y11, S1P4, sst3, VPAC2, Y2, GPR109A and S1P4, in a method of identifying a tobacco composition, such as a specific blend of tobacco leaves in a cigarette, which have increased or decreased GPCR-modulating activity, said method comprising heating or combusting a test tobacco composition to generate an aerosol and testing the aerosol for receptor-modulating activity.

In one embodiment, the present invention relates to the use of a GPCR as identified herein, particularly a GPCR selected from the group consisting of 5-HT2B, APJ, A2B, CB1, CCR10, CCR4, CGRP1, CXCR1, D1, D2, D4, EP3, EP4, ETA, GAL1, GPR40, GPR68, H1, H3, M2, MC4, mGluR2, NMU2, OPRK1, OPRM1, P2Y11, S1P4, sst3, VPAC2, Y2, GPR109A and S1P4, in a method of identifying in an aerosol produced by heating or combusting tobacco, such as cigarette smoke, particularly in the total particulate matter or the gas vapour phase of cigarette smoke, those compounds, which have GPCR-modulating activity, said method comprising fractionating the constituents of the total particulate matter or the gas vapour phase of cigarette smoke and testing the different fractions for receptor-modulating activity. Fractionation can be carried out by techniques known in the art and may include filtration and chromatography.

Once a compound or a fraction in an aerosol generated by heating or combusting tobacco, such as cigarette smoke, is identified as being active in modulating GPCR activity, the composition of a tobacco product may be altered (for example, by changing the blend of tobacco leaves) to detect a change in the amount of the compound or fraction by use of an assay based on a GPCR as identified herein.

The present invention thus provides, in another embodiment, a method for changing the composition of a tobacco product which was shown in a GPCR-based assay as identified herein of containing in an aerosol generated by heating or combusting said tobacco product compounds being active in modulating GPCR activity, which method comprises changing the blend of tobacco leaves such that the amount of these compounds in the aerosol is reduced.

In a specific embodiment of the invention, said GPCR is the GPR109A polypeptide.

In one embodiment, said receptor modulating activity is an agonistic activity.

In one embodiment, said receptor modulating activity is an antagonistic activity.

In one embodiment, said compound is present in the total particulate matter (TPM) and in the gas vapour phase (GVP) of cigarette smoke, respectively.

The invention further provides in certain embodiment methods for screening of cigarette smoke fractions or test compounds that are capable of modulating, particularly of agonizing or antagonizing, the function and/or activity of a GPCR member on the surface of a cell, particularly a cell exposed to cigarette smoke such as a cell present in lung tissue, which GPCR member is directly modulated by cigarette smoke. In a specific embodiment, said GPCR member is selected from the group consisting of 5-HT2B, APJ, A2B, CB1, CCR10, CCR4, CGRP1, CXCR1, D1, D2, D4, EP3, EP4, ETA, GAL1, GPR40, GPR68, H1, H3, M2, MC4, mGluR2, NMU2, OPRK1, OPRM1, P2Y11, S1P4, sst3, VPAC2, Y2, GPR109A and S1P4, which method comprises (i) bringing a test compound into contact with a cell or tissue expressing said GPCR and (ii) selecting those compounds which show a modulating activity on the function and/or activity of the GPCR.

In a specific embodiment of the invention, said GPCR is the GPR109A polypeptide.

In one embodiment, said receptor modulating activity is an agonistic activity.

In one embodiment, said receptor modulating activity is an antagonistic activity.

The invention further provides in certain embodiment methods for assessing smoke produced by tobacco products that modulates, particularly agonizes or antagonizes, the function and/or activity of a GPCR member on the surface of a cell, particularly a cell exposed to cigarette smoke such as a cell present in the buccal cavity which GPCR member is directly modulated by cigarette smoke. In a specific embodiment, said GPCR member is selected from the group consisting of 5-HT2B, APJ, A2B, CB1, CCR10, CCR4, CGRP1, CXCR1, D1, D2, D4, EP3, EP4, ETA, GAL1, GPR40, GPR68, H1, H3, M2, MC4, mGluR2, NMU2, OPRK1, OPRM1, P2Y11, S1P4, sst3, VPAC2, Y2, GPR109A and S1P4, which method comprises (i) contacting a cell or tissue expressing said GPCR with smoke produced by a tobacco product and (ii) measuring the modulating activity on the function and/or activity of the GPCR.

In a specific embodiment of the invention, said GPCR is the GPR109A polypeptide.

In one embodiment, said receptor modulating activity is an agonistic activity.

In one embodiment, said receptor modulating activity is an antagonistic activity.

### Definitions:

The technical terms and expressions used within the scope of this application are generally to be given the meaning commonly applied to them in the pertinent art if not otherwise indicated herein below.

As used in this specification and the appended claims, the singular forms "a", "an", and "the" include plural referents unless the context clearly dictates otherwise. Thus, for example, reference to "a compound" includes one or more compounds.

The term "modulator" as used herein is meant to include any molecule, e. g. , protein, peptide, oligopeptide, small organic molecule, chemical compound, polysaccharide, polynucleotide, etc. , having the capability to directly or indirectly alter or modify the activity or function of the GPCR polypeptide. Candidate modulatory agents or compounds or materials can also encompass numerous chemical classes, though typically they are organic molecules, preferably small organic compounds.

Modulators may comprise compounds which exhibit either agonistic or antagonistic activity towards the GPCR polypeptide.

An "antagonist" is meant herein to refer to a molecule which decreases or inhibits the amount or duration of the biological or immunological activity of the GPCR polypeptide. Antagonists can include proteins, nucleic acids, carbohydrates, antibodies, or any other molecules that decrease or reduce the effect of an RAI-3 polypeptide. A test substance is regarded as an antagonist if 50% of the activity of a known reference agonist at EC80 (effective concentration, which is expected to cause 80 % of the maximum response) were inhibited, when determined in an assay as .

An "agonist" is meant herein to refer to a molecule which increases, enhances, or prolongs the duration of the biological effect of the GPCR polypeptide. Agonists may include proteins, peptides, nucleic acids, carbohydrates, or any other molecules that bind to and modulate the effect of the GPCR polypeptide. Agonists typically enhance, increase, or augment the function or activity of an GPCR molecule.

A test substance is regarded as an agonist if it shows at least 15% of Emax of a known reference agonist.

The expression "cigarette smoke" as used herein is meant to include all kinds of aerosol or smoke generated by the heating or combustion of tobacco material, such as cigars, cigarettes, pipe tobacco, etc. Cigarette smoke may be divided into two main constituents, that is total particulate material (TPM) and a gas vapor phase (GVP). These constituents can be further fractionated by techniques known in the art for further analysis.

The particulate phase of mainstream cigarette smoke comprises between 4 and 9% of the total weight of cigarette smoke and is made up of many components including polycyclic aromatic hydrocarbons, tobacco specific nitrosamines, phenol and nicotine. Cigarette smoke total particulate matter (TPM), which excludes the gas and vapours in smoke that pass through a Cambridge filter pad on a smoking machine, is the most convenient form of cigarette smoke to extract and test. Historically, routine in vitro toxicological assessment of cigarette smoke has been conducted mainly using TPM.

Procedures for generating, collecting and extracting TPM from cigarette smoke have been established over the years as part of a CORESTA led study and have led to an ISO industry standard (ISO 4287:1991).

The terms "treatment", "treating" and the like are used herein to generally mean obtaining a desired pharmacological and/or physiological effect. The effect may be prophylactic in terms of completely or partially preventing a disease or symptom thereof and/or may be therapeutic in terms of partially or completely curing a disease and/or adverse effect attributed to the disease. The term "treatment" as used herein covers any treatment of a disease in a subject and includes: (a) preventing a disease related to an undesired immune response from occurring in a subject which may be predisposed to the disease; (b) inhibiting the disease, i.e. arresting its development; or (c) relieving the disease, i.e. causing regression of the disease.

A "patient" or "subject" or "individual" for the purposes of the present invention is used interchangeably and meant to include both humans and other animals, particularly mammals, and other organisms. Thus, the methods are applicable to both human therapy and veterinary applications. In the preferred embodiment the patient or subject is a mammal, and in the most preferred embodiment the patient or subject is a human.

The expressions "pharmaceutical composition" and "therapeutical composition" are used herein interchangeably in the widest sense. They are meant to refer, for the purposes of the present invention, to a therapeutically effective amount of the active ingredient, i.e. a modulator of GPCR function and/or activity or a pharmaceutically acceptable salt thereof, optionally, together with a pharmaceutically acceptable carrier or diluent. It embraces compositions that are suitable for the curative treatment, the control, the amelioration, an improvement of the condition or the prevention of a disease or disorder in a human being or a non-human animal. Thus, it embraces pharmaceutical compositions for the use in the area of human or veterinary medicine. Such a "therapeutic composition" is characterized in that it embraces at least one modulator of GPCR function and/or activity or a physiologically acceptable salt thereof, and optionally a carrier or excipient whereby the salt and the carrier and excipient are tolerated by the target organism that is treated therewith.

The term "pharmaceutically acceptable salts" include salts of acidic or basic groups present in compounds of the invention. Pharmaceutically acceptable acid addition salts include, but are not limited to, hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzensulfonate, p-toluenesulfonate and pamoate (i.e., 1,1'-methylene-bis-(2-hydroxy-3-naphthoate)) salts. Certain compounds of the invention can form pharmaceutically acceptable salts with various amino acids. Suitable base salts include, but are not limited to, aluminum, calcium, lithium, magnesium, potassium, sodium, zinc, and diethanolamine salts.

A "therapeutically effective amount" refers to that amount which provides a therapeutic effect for a given condition and administration regimen. In particular, "therapeutically effective amount" means an amount that is effective to prevent, alleviate or ameliorate symptoms of the disease or prolong the survival of the subject being treated, which may be a human or non-human animal. Determination of a therapeutically effective amount is within the skill of the person skilled in the art.

The therapeutically effective amount or dosage of a compound according to this invention can vary within wide limits and may be determined in a manner known in the relevant art. The dosage can vary within wide limits and will, of course, have to be adjusted to the individual requirements in each particular case.

In one embodiment of the invention, methods are provided for screening of cigarette smoke fractions or test compounds that are capable of modulating, particularly of agonizing or antagonizing, the function and/or activity of a GPCR, which is directly modulated by cigarette smoke, particularly a GPCR selected from the group consisting of 5-HT2B, APJ, A2B, CB1, CCR10, CCR4, CGRP1, CXCR1, D1, D2, D4, EP3, EP4, ETA, GAL1, GPR40, GPR68, H1, H3, M2, MC4, mGluR2, NMU2, OPRK1, OPRM1, P2Y11, S1P4, sst3, VPAC2, Y2, GPR109A and S1P4, which method comprises (i) bringing a cigarette smoke fraction or a test compound into contact with a cell or tissue expressing said GPCR and (ii) selecting those fractions or compounds which show a modulating activity on the function and/or activity of the GPCR.

In a specific embodiment of the invention, said GPCR is GPR109A.

For identification of active fractions in cigarette smoke or compounds that are capable of binding a GPCR directly modulated by cigarette smoke, particularly the GPR109A polypeptide, *in vitro* assays may be used which are known to those skilled in the art.

In these assays a reaction mixture comprising the GPCR polypeptide and the cigarette smoke fraction or test compound is provided under conditions and for a time sufficient to allow the two components to interact and bind, thus forming a complex which can be removed and/or detected in the reaction mixture. There exist a multitude of different designs that may be applied in such an in vitro assay. For example, one method to conduct such an assay would involve expressing the GPCR polypeptide by recombinant DNA technology in a host cell line that express an endogenous promiscuous Gα15/16 to provide a general calcium readout which is independent of the ligand activating the GPCR.

The compound identified in said assays may be used, particularly in form of a pharmaceutical composition, to modulate, e.g. agonize or antagonize, the function and/or activity of the GPCR polypeptide by disrupting normal GPCR polypeptide interactions with the receptors native ligands. The cigarette smoke fraction identified in said assays may be further investigated to isolate the one or more compounds that directly modulate the GPCR. The assay disclosed herein can be used to confirm if the compound(s) in an active fraction are responsible for direct modulation of the GPCR. As GPCR is known to play a role in odour perception, the assay disclosed herein can also be used to assess different blends of tobacco leaves which produce a greater or lesser amount of the compound(s) that directly modulate the GPCR, thereby aiding the blending of tobacco leaves in manufacturing cigarettes.

Administration of suitable (pharmaceutical) compositions containing the active ingredient according to the invention and as disclosed herein, may be effected by different ways known to a person skilled in the art, e.g., by parenteral, subcutaneous, intraperitoneal, topical, transmucosal, transdermal, intrabronchial, intrapulmonary and intranasal, intraventricular, intravaginal administration and, if desired for local treatment, intralesional administration. Parenteral administrations include intraperitoneal, intramuscular, intradermal, subcutaneous, intravenous or intraarterial administration. The compositions of the invention may also be administered directly to the target site, e.g., by biolistic delivery to an external or internal target site, like a specifically effected organ or a tumor.

The compounds of the present invention and the pharmaceutical compositions containing said compounds, may be administered orally, and thus be formulated in a form suitable for oral administration, i.e. as a solid or a liquid preparation. Suitable solid oral formulations include tablets, capsules, pills, granules, pellets and the like. Suitable liquid oral formulations include solutions, suspensions, dispersions, emulsions, oils and the like. If formulated in form of a capsule, the compositions of the present invention comprise, in addition to the active compound and the inert carrier or diluent, a hard gelating capsule.

Examples of suitable pharmaceutical carriers, excipients and/or diluents are well known in the art and include, but are not limited to, a gum, a starch (e.g. corn starch, pregeletanized starch), a sugar (e.g., lactose, mannitol, sucrose, dextrose), a cellulosic material (e.g. microcrystalline cellulose), an acrylate (e.g. polymethylacrylate), calcium carbonate, magnesium oxide, talc, or mixtures thereof.

Pharmaceutically acceptable carriers for liquid formulations are aqueous or non-aqueous solutions, suspensions, emulsions or oils. Examples of non-aqueous solvents are propylene glycol, polyethylene glycol, and injectable organic esters such as ethyl oleate. Examples of oils are those of animal, vegetable, or synthetic origin, for example, peanut oil, soybean oil, olive oil, sunflower oil, fish-liver oil, another marine oil, or a lipid from milk or eggs.

Aqueous carriers include water, alcoholic/aqueous solutions, emulsions or suspensions, including saline and buffered media such as phosphate buffered saline solutions, water, emulsions, such as oil/water emulsions, various types of wetting agents, sterile solutions etc. Compositions comprising such carriers can be formulated by well known conventional methods. Suitable carriers may comprise any material which, when combined with the biologically active compound of the invention, retains the biological activity.

Preparations for parenteral administration may include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles may include sodium chloride solution, Ringer's dextrose, dextrose and sodium chloride, lactated Ringer's, or fixed oils. Intravenous vehicles may include fluid and nutrient replenishes, electrolyte replenishers (such as those based on Ringer's dextrose), and the like. Preservatives and other additives may also be present including, for example, antimicrobials, anti-oxidants, chelating agents, and inert gases and the like. In addition, the pharmaceutical composition of the present invention might comprise proteinaceous carriers, like, e.g., serum albumin or immunoglobulin, preferably of human origin.

The pharmaceutical compositions provided herein may also be administered as controlled-release compositions, i.e. compositions in which the active ingredient is released over a period of time after administration. Controlled- or sustained-release compositions include formulation in lipophilic depots (e.g. fatty acids, waxes, oils). In another embodiment, the composition is an immediate-release composition, i.e. a composition in which all the active ingredient is released immediately after administration.

Further, a modulator of GPCR function and/or activity according to the invention and as described herein or a composition comprising said modulator may be administered admixed to food, functional food, drinks, or medicinal food. These pharmaceutical compositions can be administered to the subject at a suitable dose. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently.

The total daily dosages of a modulator of GPCR function and/or activity employed in both veterinary and human medicine will suitably be in the range 0,01-2000 mg/kg body-weight, preferably from 0,1-1000 mg/kg body-weight, preferably from 1-100 mg/kg and these may be administered as single or divided doses, and in addition, the upper limit can also be exceeded when this is found to be indicated. Such dosage will be adjusted to the individual requirements in each particular case including the specific compound(s) being administered, the route of administration, the condition being treated, as well as the patient being treated. However, the compounds can also be administered as depot preparations (implants, slow-release formulations, etc.) weekly, monthly or at even longer intervals. In such cases the dosage will be much higher than the daily one and has to be adapted to the administration form, the body weight and the concrete indication. The appropriate dosage can be determined by conducting conventional model tests, preferably animal models. In general, in the case of oral or parenteral administration to adult humans weighing approximately 70 kg, a daily dosage of about 10 mg to about 10.000 mg, preferably from about 200 mg to about 1.000 mg, should be appropriate, although the upper limit may be exceeded when indicated. The daily dosage can be administered as a single dose or in divided doses, or for parenteral administration; it may be given as continuous infusion.

An effective dose of active ingredient(s) depends at least on the nature of the condition being treated, toxicity, whether the compound(s) is being used prophylactically (lower doses) or against an active infection or condition, the method of delivery, and the pharmaceutical formulation, and will be determined by the clinician using conventional dose escalation studies. It can be expected to be from about 0.05 to about 30 mg/kg body weight per day. For example, for topical delivery the daily candidate dose for an adult human of approximately 70 kg body weight will range from about 1 mg to about 500 mg, generally between about 5 mg and about 40 mg, and may take the form of single or multiple doses or administration sites.

The pharmaceutical composition according to the invention comprising a compound capable of modulating the function and/or activity of a GPCR which is directly modulated by cigarette smoke, particularly a GPCR selected from the group consisting of 5-HT2B, APJ, A2B, CB1, CCR10, CCR4, CGRP1, CXCR1, D1, D2, D4, EP3, EP4, ETA, GAL1, GPR40, GPR68, H1, H3, M2, MC4, mGluR2, NMU2, OPRK1, OPRM1, P2Y11, S1P4, sst3, VPAC2, Y2, GPR109A and S1P4, may be used in methods for studying the effects associated with or induced by exposure to cigarette smoke in an individual with the aim of developing means for the treatment, prevention or alleviation of a disease, disorder or condition associated with or induced by exposure to cigarette smoke.

In particular, said disease, disorder or condition are selected from the group consisting of lung diseases such as chronic obstructive pulmonary disease (COPD) (including emphysema and chronic bronchitis), heart diseases such as coronary artery disease, heart attacks, strokes, and cancer, particularly acute myeloid leukemia; bladder cancer, cancer of the cervix, cancer of the esophagus, kidney cancer, cancer of the larynx (voice box), lung cancer, cancer of the oral cavity (mouth), cancer of the pharynx (throat), stomach cancer, cancer of the uterus, and peripheral vascular diseases and hypertension, and arteriosclerosis.

The foregoing description will be more fully understood with the reference to the following Examples. Such Examples are, however, exemplary methods of practicing the present invention and are not intended to limit the scope of the invention.

### Example 1: Materials and Methods

### 1.1 Assay Design

Mainstream smoke (MS) from the reference cigarette 3R4F was screened for its potential to interact with 151 GPCRs. The ChemiScreen™ system developed at Millipore for the detection and pharmacological characterization of agonistic or antagonistic activities for GPCRs was used. Briefly, recombinant GPCRs are expressed in host cell lines that express endogenous promiscuous Gα15/16 to provide a general calcium readout system independent of the ligand activating the respective GPCR. After ligand binding, the Gα15/16 activates phospholipase β leading to the formation of inositoltrisphosphat (IP3). After binding of IP3 to the intracellular ryanodine receptor, Ca2+ is released from the endoplasmatic reticulum (ER). The change of the intracellular calcium concentrations was determined using the FLIPRTETRA (Molecular Devices, Sunnyvale, USA). A 'two-addition' protocol is used to screen MS for agonistic or antagonistic activity, in which the first addition is the TPM or GVP and the second addition is an EC80 (effective concentration, which is expected to cause 80 % of the maximum response) amount of a known agonist. A test substance is regarded as an agonist if it shows at least 15% of Emax of a known reference agonist. A test substance is regarded as an antagonist if 50% of the activity of a known reference agonist at EC80 were inhibited.

### 1.2 Compound preparation master stock solution

Unless specified otherwise, TPM or gas vapor phase (GVP) samples were diluted in 100% anhydrous dimethyl sulfoxide (DMSO) including all dilutions. If the cigarette smoke fraction is provided in a different solvent, all master stock dilutions were prepared in the specified solvent. All control wells contained identical final concentrations of the solvent as the wells containing cigarette smoke fractions.

### 1.3 Compound plate for assay

GVP and TPM were transferred from a master stock solution into a 'daughter' plate that was used in the assay. Each sample compound was diluted into assay buffer (1 x Hank's Buffered Salt Solution (HBSS) with 20 mM 2-(4-(2-Hydroxyethyl) - 1-piperazinyl)-ethansulfonsäure (HEPES) and 2.5 mM Probenecid) at an appropriate concentration to obtain final concentrations (TPM: 0.1 mg/ml, 0.03 mg/ml, 0.01 mg/ml; GVP: 0.1 puffs/ml, 0.03 puffs/ml, 0.01 puffs/ml).

### 1.4 Calcium flux assay

### 1.4.1 Agonist assay format

Agonist activity was determined by assaying TPM and GVP on each GPCR. As a control, the Emax of a known agonist of each GPCR was used. The TPM or GVP samples were pipetted in duplicate at concentrations specified above and determined for a change of intracellular calcium concentration for 180 s using the FLIPRTETRA. At the completion of the first assay run, the assay plate was removed from the FLIPRTETRA and placed at 25 °C for seven minutes in order to ensure a complete recovery of the intracellular calcium stores.

### 1.4.2 Antagonist assay format

All pre-incubated sample compounds and reference agonists containing wells were stimulated with a concentration of EC80 of the reference agonist. Fluorescent measurements were taken for 180 s using the FLIPRTETRA to calculate IC50 values.

### 1.5 Data processing

All plates were subjected to appropriate baseline corrections. Once baseline corrections were processed, maximum fluorescence values were exported and data manipulated to calculate percentage activation, percentage inhibition and Z'.

### 1.6 QC Criteria

| | |
|---|---|
| Percent Effect Validation *: | Duplicates must be <30% divergent |
| Z' Statistic *†*: | Must be >0.5 |
| Signal-to-Noise (S/N) *†*: | Must be >5 |
| R2 *†*: | Must be >0.90 |
| IC50 Value for reference antagonist(s) *†*: | Must be within 10-fold of historic EC50 |
| value | |

| | |
|---|---|
| * If these QC conditions fail, that concentration is removed from curve fitting. If two or more concentrations within the dose response fail, the entire compound curve of that compound is repeated on a different assay plate. † If any of these QC conditions fail, all data collected from that particular GPCR are repeated. | |

### References

Im, D. S., Orphan G protein-coupled receptors and beyond. Jpn J Pharmacol; 90: 101-6 (2002)
Kostenis, E. A., Glance at G-protein-coupled receptors for lipid mediators: a growing receptor family with remarkably diverse ligands. Pharmacol Ther; 102: 243-57 (2004)
Knowles, H. J., te Poele, R. H., Workman, P., Harris, A. L., Niacin induces PPARgamma expression and transcriptional activation in macrophages via HM74 and HM74a-mediated induction of prostaglandin synthesis pathways. Biochem Pharmacol.; 71 (5): 646-56. (2006)
Lättig, J. H., Investigations of interactions of G-protein-coupled receptors with their ligands and G-proteins based on homologous receptor subtypes with different biological functions. Dissertation, Free University of Berlin (2007)
Lorenzen, A., Stannek, C., Burmeister, A., Kalvinsh, I., Schwabe, U., G-protein coupled receptor for nicotinic acid in mouse macrophages. Biochem Pharmacol 7375:1-4 (2002)
Maciejewski-Lenoir, D., Richman, J. G., Hakak, Y., Gaidarov, I., Behan, D. P., Connolly, D. T., Langerhans cells release prostaglandin D2 in response to nicotinic acid. J. Invest. Dermatol. 126:2637-46 (2006)
PMRL study P 11702, Targeted GC-MS Fingerprinting of Mainstream Smoke from the PMI Smoking Article SMAR, the Marlboro 6 mg, and the Reference Cigarette 3R4F Smoked Under Different Smoking Regimens Study director: A. Wittig Date of report: 16 Jan.2009
Perez, D. M., From plants to man: the GPCR "tree of life". Mol Pharmacol; 67: 1383-4 (2005)
Schaub A., Futterer A., Pfeffer K., PUMA-G, an IFN-gamma-inducible gene in macrophages is a novel member of the seven transmembrane spanning receptor superfamily. Eur J Immunol.; 31 (12): 3714-25. (2001)
Shen, H. C., Ding, F. X., Luell, S., Forrest, M. J., Carballo-Jane, E., Wu, K. K, Wu, T. J, Cheng, K., Wilsie, L. C, Krsmanovic, M. L., Taggart, A. K., Ren, N., Cai, T. Q., Deng, Q., Chen. Q., Wang, J., Wolff, M. S., Tong, X., Holt, T. G., Waters, M. G., Hammond, M. L., Tata, J. R., Colletti, S. L., Discovery of biaryl anthranilides as full agonists for the high affinity niacin receptor. J Med Chem.; 50 (25): 6303-6 (2007)
Szanto, A., Roszer, T., Nuclear receptors in macrophages: a link between metabolism and inflammation. FEBS Lett.; 582(1): 106-16 (2008)
Taggart, A. K., Kero, J., Gan, X., Cai, T. Q., Cheng, K., Ippolito, M., Ren, N., Kaplan, R., Wu, K., Wu, T. J., Jin, L., Liaw, C., Chen, R., Richman, J., Connolly, D., Offermanns, S., Wright, S. D., Waters, M. G., (D)-beta-Hydroxybutyrate inhibits adipocyte lipolysis via the nicotinic acid receptor PUMA-G. J Biol Chem.; 280 (29): 26649-52 (2005)
Tunaru, S., Kero, J., Schaub, A., Wufka, C., Blaukat, A., Pfeffer, K., Offermanns, S., PUMA-G and HM74 are receptors for nicotinic acid and mediate its anti-lipolytic effect. Nat Med.; 9 (3): 352- 5.(2003)
Tunaru, S., Lättig, J., Kero, J., Krause, G., Offermanns S. Characterization of determinants of ligand binding to the nicotinic acid receptor GPR109A (HM74A/PUMA-G). Mol Pharmacol.;68 (5): 1271-80. (2005)
Vassilatis, D. K" Hohmann, J. G" Zeng, H., Li, F., Ranchalis, J. E., Mortrud, M. T" et al. The G protein-coupled receptor repertoires of human and mouse. Proc Natl Acad Sci USA; 100: 4903-8 (2003)
Wise, A., Foord, S. M" Fraser, N. J., Barnes, A. A., Elshourbagy, N., Eilert, M., Ignar, D. M., Murdock, P. R., Steplewski, K., Green, A., Brown, A. J., Dowell, S. J., Szekeres, P. G., Hassall, D. G., Marshall, F. H., Wilson, S., Pike, N. B., Molecular identification of high and low affinity receptors for nicotinic acid. J Biol Chem.; 278 (11): 9869-74 (2003)
Whitey, G. S., Opiteck, G., Garulacan, L. A., Ramanathan, C. S., McKinnon, M., Bennett, K. L, Barber, L. E., Cacae, A., Tsuchihashi, Z., Identification and modulation of a G-protein coupled receptor (GPCR), RAI3, associated with chronic obstructive pulmonary disease (COPD) and NF-KB and E-selectin regulation. International Patent Application Pub. No. WO 2004/001060, 31. December 2003
Yousefi, S., Cooper, P. R., Mueck, B., Potter, S. L., Jarai, G., cDNA representational difference analysis of human neutrophils stimulated by GM-CSF. Biochem. Biophys. Res. Common 277:401-9 (2000)

### Abbreviations *

CRO: Contract Research Organization
DMSO: dimethyl sulfoxide
ER: endoplasmatic reticulum
GPCR: G-protein coupled receptors
GVP: gas vapor phase
ISO: International Organization for Standardization
LR: Language Center and Reporting
MS: mainstream smoke
TPM: total particulate matter

## Claims

1. A method for regulating signal transduction pathways activated by cigarette smoke by modulating the function and/or activity of a GPCR member on the cell surface, which is directly modulated by cigarette smoke.

2. The method of claim 1, wherein said GPCR member is selected from the group consisting of 5-HT2B, APJ, A2B, CB1, CCR10, CCR4, CGRP1, CXCR1, D1, D2, D4, EP3, EP4, ETA, GAL1, GPR40, GPR68, H1, H3, M2, MC4, mGluR2, NMU2, OPRK1, OPRM1, P2Y11, S1P4, sst3, VPAC2, Y2, GPR109A and S1 P4.

3. The method of claim 2, wherein said GPCR member is the GPR109A polypeptide.

4. The method of any one of claims 1 - 3, wherein said signal transduction pathway is the prostaglandin synthesis pathway.

5. A method for screening of candidate compounds that are capable of modulating, particularly of agonizing or antagonizing, the function and/or activity of a GPCR member on the cell surface, which is directly modulated by cigarette smoke.

6. The method of claim 5, wherein said GPCR member is selected from the group consisting of 5-HT2B, APJ, A2B, CB1, CCR10, CCR4, CGRP1, CXCR1, D1, D2, D4, EP3, EP4, ETA, GAL1, GPR40, GPR68, H1, H3, M2, MC4, mGluR2, NMU2, OPRK1, OPRM1, P2Y11, S1P4, sst3, VPAC2, Y2, GPR109A and S1P4.

7. The method of claim 6, wherein said GPCR member is the GPR109A polypeptide.

8. A compound capable of modulating, particularly of agonizing or antagonizing, the function and/or activity of a GPCR member on the cell surface, which is directly modulated by cigarette smoke, for use in a method of studying the effects associated with or induced by exposure to cigarette smoke in an individual.

9. The compound of claim 8, wherein said GPCR member is selected from the group consisting of 5-HT2B, APJ, A2B, CB1, CCR10, CCR4, CGRP1, CXCR1, D1, D2, D4, EP3, EP4, ETA, GAL1, GPR40, GPR68, H1, H3, M2, MC4, mGluR2, NMU2, OPRK1, OPRM1, P2Y11, S1P4, sst3, VPAC2, Y2, GPR109A and S1P4.

10. The compound of claim 9, wherein said GPCR member is the GPR109A polypeptide.

11. A method for assessing cigarette smoke or fractions thereof that are capable of modulating, particularly of agonizing or antagonizing, the function and/or activity of a GPCR member on the cell surface, which is directly modulated by cigarette smoke.

12. The method of claim 11, wherein said compound is an antagonist of GPCR function.

13. The method of claim11, wherein said compound is an agonist of GPCR function.

14. The method of claim 12, wherein said GPCR member is the GPR109A polypeptide.

15. Use of a GCPR which is directly modulated by cigarette smoke, particularly a GPCR member selected from the group consisting of 5-HT2B, APJ, A2B, CB1, CCR10, CCR4, CGRP1, CXCR1, D1, D2, D4, EP3, EP4, ETA, GAL1, GPR40, GPR68, H1, H3, M2, MC4, mGluR2, NMU2, OPRK1, OPRM1, P2Y11, S1P4, sst3, VPAC2, Y2, GPR109A and S1P4, particularly GPR109A, in a method of identifying in cigarette smoke those compounds or fractions, which have receptor-modulating activity.
